# EUROPEAN PATENT SPECIFICATION

(11) **EP 2 376 410 B1**
(45) Date of publication and mention of the grant of the patent: **29.08.2018**
(21) Application number: 09837945.6
(22) Date of filing: 17.12.2009
(51) Int. Cl.: C11D 7/50, C11D 3/43, C11D 1/00, C23G 5/028

(54) **Method for drying**
Trockungsverfahren
Méthode pour séchage

(30) Priority: 17.12.2008 US 138164 P
(43) Date of publication of application: 19.10.2011
(73) Proprietor: Honeywell International Inc., Morris Plains, NJ 07950 (US)
(72) Inventor: BASU, Rajat, East Amherst NY 14051 (US); ZYHOWSKI, Gary, Lancaster NY 14068 (US); COOK, Kane, Eggertsville NY 14226 (US); NALEWAJEK, David, West Seneca NY 14221 (US)
(74) Representative: Crooks, Elizabeth Caroline
(86) International application number: PCT/US2009/068588
(87) International publication number: WO 2010/080544

(56) References cited:
- WO-A2-2007/053673
- WO-A2-2009/140231
- US-A- 5 610 128
- US-A- 5 856 286
- US-A- 5 868 799
- US-A1- 2006 142 173
- US-A1- 2008 255 260
- DATABASE WPI Week 199042 Thomson Scientific, London, GB; AN 1990-315370 XP002728883, & JP H02 222496 A (ASAHI GLASS CO LTD) 5 September 1990 (1990-09-05)

## Description

### FIELD OF THE INVENTION

The invention relates to methods for drying the surface of a substrate.

### BACKGROUND OF THE INVENTION

The use of aqueous compositions for the surface treatment of metal, ceramic, glass, and plastic articles is well known. Additionally, cleaning, plating, and deposition of coatings on the surface of articles are known to be carried out in aqueous media. In both cases, a halocarbon solvent and a hydrophobic surfactant may be used to displace water from a water-laden surface.

A variety of solvent-surfactant drying compositions for water displacement have been utilized. For example, solvent-surfactant compositions based on 1,1,2trichlorotrifluoroethane ("CFC-113") are known. However, environmental concerns are leading to a decline in the use of such CFC-based systems.

Applicants have come to recognize that it is generally not possible to predict whether a particular surfactant or group of surfactants will be fully acceptable for use with a given solvent or group of solvents, including hydrochlorofluorocarbons ("HCFC's"), hydrofluorocarbons ("HFC's"), hydrochlorofluoro-olefins ("HCFO's") and hydrofluoro-olefins ("HFO's") and hydrofluoroethers ("HFE's") solvents suitable to replace CFC solvents. Moreover, many of the known surfactants cannot be dissolved in such solvents. Further, dry cleaning, drying, and water displacement require surfactants that, together with the chosen solvent, impart distinct, and a difficult to achieve set of properties to the cleaning compositions. For the removal of oil from machined parts, the surfactant will preferably aid in the removal of the soils that would otherwise only be sparingly soluble in such solvents. Additionally, water displacement requires a surfactant that does not cause the formation a stable emulsion with water.

Applicants have come to appreciate that halogenated olefin solvents in general, and chloro-fluoro-olefins in particular, present the additional difficulty of identifying combinations of such solvents and surfactants that not only possess the desired solvency and other properties, but which also exhibit an acceptable level of stability since olefins are generally understood to be reactive, especially in comparison to many previously used solvents.

Therefore, one must not only identify those surfactants soluble in the HCFC, HFC, HCFO, HFO, or HFE solvent selected, but also surfactants that also have the desired activity in the solvents and which exhibit acceptable levels of stability.

US2006/0142173 discloses the use of fluoroalkenes such as HFO-1214, HFO-1233 or HFO-1354 in solvent cleaning applications.

### SUMMARY OF THE INVENTION

The present invention relates to method for drying the surface of a substrate comprising the steps of contacting the substrate with a composition comprising 1-chloro-3,3,3-trifluoropropene (HFCO-1233zd) and at least one second solvent selected from alcohols of the group consisting of methanol, ethanol or isopropanol, and then removing the composition from the substrate; wherein the alcohol is present in an amount of from 1 to 50 weight percent based on the total composition and 1-chloro-3,3,3-trifluoropropene is present in the amount of at least 50 weight percent based upon the total weight of solvent present in the composition.

The present invention is directed to compositions containing 1-chloro-3,3,3-trifluoropropene with at least one alcohol, in particular methanol, ethanol, or isopropanol
and the use of the compositions disclosed herein for drying.

### DETAILED DESCRIPTION OF THE INVENTION

The preferred compositions of the invention effectively displace water from a broad range of substrates including, without limitation: metals, such as stainless steel, aluminum alloys, and brass; glass and ceramic surfaces, such as glass, borosilicate glass and unglazed alumina; silica, such as silicon wafers; fired alumina; and the like. Further, the compositions of the invention either do not form noticeable emulsions with the displaced water or form only insignificant amounts of such emulsions.

The term HFCO-1233zd is used herein generically to refer to 1,1,1-trifluo-3,chloro-propene, independent of whether it is the cis- or trans- form. The terms "cisHFC0-1233zd" and "transHFC0-1233zd" are used herein to describe the cis- and trans- forms of 1, 1, 1-trifluo,3-chlororopropene, respectively. The term "HFCO-1233zd" therefore includes within its scope cisHFC0- 1233zd, transHFC0-1233zd, and all combinations and mixtures of these.

In certain embodiments, the solvent portion of the composition comprises, in an amount of at least about 50% by weight based on the weight of the total weight of the solvent in the compositon, HCF0-1233zd, and even more preferably transHCF0-1233zd.

The compositions of the invention may be used to clean and/or dry nonabsorbent substrates and articles constructed of such materials as metals, glasses, ceramics, and the like. Thus, the invention provides a method for drying the surface of a substrate comprising the steps of contacting the substrate with a composition comprising a solvent comprising a 1-chloro-3,3,3-trifluoropropene and an alcohol then removing the solvent-surfactant composition from the article.

The manner of contacting is not critical and may vary widely. For example, the article may be immersed in a container of the composition or the article may be sprayed with the composition. Complete immersion of the article is preferred because it ensures contact between all exposed surfaces of the article and the composition. Any method that can provide such contact may be used. Typically, the contacting time is up to about 10 minutes, but this time is not critical and longer times may be used if desired.

The contacting temperature may also vary widely depending on the boiling point of the compositions. In general, the temperature is equal to or less than about such boiling point. Following the contacting step, the article is removed from contact with the composition and removal of composition adhering to exposed surfaces of the article is effected by any conventional means such as evaporation. Optionally, the remaining minimal amounts of surfactant adhering to the article may be removed further by contacting the article with surfactant free solvent that is hot or cold. Finally, holding the article in the solvent vapor will decrease further the presence of the surfactant residue remaining on the article. Again, removal of solvent adhering to the article is effected by evaporation.

In general, removal, or evaporation, of the composition is effected in less than about 30 seconds, preferably less than about 10 seconds. Neither temperature nor pressure is critical. Atmospheric or sub-atmospheric pressure may be employed and temperatures above and below the boiling point of the hydrochlorofluoro-olefin or hydrofluoroolefin may be used. Optionally, additional surfactants may be included in the overall composition as desired.

A second solvent is included which is an alcohol. This solvent is methanol, ethanol, or isopropanol.

The alcohol is present in amounts of from 1 to 50 weight percent, preferably from 4 to 45 percent, based on the total composition.

The compositions and processes of the invention are preferably carried out or used with conventional drying machines and systems. Illustrative of such drying machines are those described in U.S. Pat. No. 3,386,181.

### EXAMPLES

### Reference Example 1

The performance of the solvent-surfactant composition of the invention in the displacement of water was evaluated by placing 35 mL of the solvent 1-chloro3,3,3trifluoro- 1-propene containing 500 ppm by weight of octylphenyl acid phosphate salt of perfluoroalkyl prydinium (the surfactant prepared in Example 2 of 5,856,286) in a 100 mL beaker fitted with a cooling coil. The solution was brought to a boil whereby the coiling coil confined the solvent vapor to the beaker. Duplicate 316 stainless steel coupons, wet-abraded to a water-break-free condition, were immersed in water and then into the boiling sample solution. The time required to displace the water from the coupon was recorded, a minimum observation time of 5 second was chosen.

After an initial observation of drying performance, 35 mL of water was added to the boiling solution. The solution was kept boiling for 5 minutes in order to provide contact between the solution and the water. The mixture was then transferred to a separatory funnel and the time for phase separation was noted. Rapid separation into clear phases with no emulsion layer was an indication that the solution will perform successfully in the application. A clear water phase indicated that no gross loss of drying solvent to the water effluent of a commercial machine would be expected. In this test, a clear solvent phase indicated the ability of the drying solvent to expel displaced water from a drying machine in a particle time frame, i.e., water will not accumulate in the solvent phase.

The results demonstrated that the octylphenyl acid phosphate salt of perfluoroalkyl prydinium telomer performed as an active surfactant for water displacement with respect to drying time, phase separation, phase clarity, and for HFCO solvent at the 500 ppm level.

### Example 2

The performance of a solvent-solvent composition of the invention in the displacement of water is evaluated by placing 35 mL of 1-chloro-3,3,3-trifluorolpropene solvent containing 5% by weight methanol solvent in a 100 mL beaker fitted with a cooling coil. The solution is brought to a boil whereby the coiling coilconfines the solvent vapor to the beaker. Duplicate 316 stainless steel coupons, wetabraded to a water-break-free condition, are immersed in water and then into the boiling sample solution. The time required to displace the water from the coupon is recorded using a minimum observation time of 5 seconds. The solvent alcohol blend could remove water completely from the substrate.

## Claims

1. A method for drying the surface of a substrate comprising the steps of contacting the substrate with a composition comprising 1-chloro-3,3,3-trifluoropropene (HFCO-1233zd) and at least one second solvent selected from alcohols of the group consisting of methanol, ethanol or isopropanol, and then removing the composition from the substrate;
wherein the alcohol is present in an amount of from 1 to 50 weight percent based on the total composition and 1-chloro-3,3,3-trifluoropropene is present in the amount of at least 50 weight percent based upon the total weight of solvent present in the composition.

2. The method as claimed in claim 1, wherein the alcohol is present in an amount of from 1 to 45 weight percent based on the total composition.

3. The method as claimed in claim 2, wherein the alcohol is present in an amount of from 1 to 4 weight percent based on the total composition.

4. The method as claimed in any preceding claim wherein the 1-chloro-3,3,3-trifluoropropene (HFCO-1233zd) comprises cis HFCO-1233zd, trans HFCO-1233zd or a mixture thereof.

5. The method as claimed in any preceding claim wherein the 1-chloro-3,3,3-trifluoropropene comprises trans HFCO-1233zd.

6. The method as claimed in any of claims 1 to 4, wherein the 1-chloro-3,3,3-trifluoropropene comprises cis HFCO-1233zd.

7. The method as claimed in any preceding claim, wherein the at least one second solvent selected from alcohols is methanol.

8. The method as claimed in claim 1 for drying metals, glass and ceramic surfaces, silica and/or fired alumina.

9. The method of any of claim 1, wherein the composition additionally comprises a tetrafluropropene.

10. The method of claim 8, wherein the composition is provided for displacing water from stainless steel, aluminium alloys and brass.

11. The method of claim 8, wherein the composition is provided for displacing water from glass or borosilicate glass

12. The method of claim 8, wherein the composition is provided for displacing water from silicon wafers.

13. A method as claimed in any of claims 1 to 12 for drying a non-absorbent substrate.

14. A method as claimed in any preceding claim wherein the substrate is contacted with the composition for up to 10 minutes.

15. A method as claimed in any preceding claim, wherein the substrate is contacted with the composition at a temperature equal to or less than the boiling point of the composition.

16. A method as claimed in any preceding claim, wherein the substrate is sprayed with the composition.

17. A method as claimed in any preceding claim, wherein the substrate is immersed in the composition.

18. A method of claim 24, wherein the substrate is completely immersed in the composition.

19. A method as claimed in any preceding claim, wherein any composition adhering to exposed surfaces of the substrate is removed by evaporation.

20. A method as claimed in claim 19, wherein the composition is removed by evaporation in less than 30 seconds.

21. A method as claimed in claim 19 or claim 20, wherein the evaporation is carried out at atmospheric or sub atmospheric pressure.

## Patentansprüche

1. Verfahren zum Trocknen der Oberfläche eines Substrats, bei dem man das Substrat mit einer Zusammensetzung, die 1-Chlor-3,3,3-trifluorpropen (HFCO-1233zd) und mindestens ein zweites Lösungsmittel, das aus Alkoholen der Gruppe bestehend aus Methanol, Ethanol oder Isopropanol ausgewählt ist, umfasst, in Berührung bringt und dann die Zusammensetzung von dem Substrat entfernt;
wobei der Alkohol in einer Menge von 1 bis 50 Gew.-%, bezogen auf die gesamte Zusammensetzung, vorliegt und 1-Chlor-3,3,3-trifluorpropen in einer Menge von mindestens 50 Gewichtsprozent, bezogen auf das Gesamtgewicht von in der Zusammensetzung vorliegendem Lösungsmittel, vorliegt.

2. Verfahren nach Anspruch 1, bei dem der Alkohol in einer Menge von 1 bis 45 Gew.-%, bezogen auf die gesamte Zusammensetzung, vorliegt.

3. Verfahren nach Anspruch 2, bei dem der Alkohol in einer Menge von 1 bis 4 Gew.-%, bezogen auf die gesamte Zusammensetzung, vorliegt.

4. Verfahren nach einem der vorhergehenden Ansprüche, bei dem das 1-Chlor-3,3,3-trifluorpropen (HFCO-1233zd) cis-HFCO-1233zd, trans-HFCO-1233zd oder eine Mischung davon umfasst.

5. Verfahren nach einem der vorhergehenden Ansprüche, bei dem das 1-Chlor-3,3,3-trifluorpropen trans-HFCO-1233zd umfasst.

6. Verfahren nach einem der Ansprüche 1 bis 4, bei dem das 1-Chlor-3,3,3-trifluorpropen cis-HFCO-1233zd umfasst.

7. Verfahren nach einem der vorhergehenden Ansprüche, bei dem es sich bei dem aus Alkoholen ausgewählt und zweiten Lösungsmittel um Methanol handelt.

8. Verfahren nach Anspruch 1 zum Trocknen von Metallen, Glas- und Keramikoberflächen, Siliciumdioxid und/oder gebranntem Aluminiumoxid.

9. Verfahren nach Anspruch 1, wobei die Zusammensetzung zusätzlich ein Tetrafluorpropen umfasst.

10. Verfahren nach Anspruch 8, bei dem die Zusammensetzung zum Verdrängen von Wasser von nichtrostendem Stahl, Aluminiumlegierungen und Messing bereitgestellt wird.

11. Verfahren nach Anspruch 8, bei dem die Zusammensetzung zum Verdrängen von Wasser von Glas oder Borosilikatglas bereitgestellt wird.

12. Verfahren nach Anspruch 8, bei dem die Zusammensetzung zum Verdrängen von Wasser von Silicium-Wafern bereitgestellt wird.

13. Verfahren nach einem der Ansprüche 1 bis 12 zum Trocknen eines nicht absorptionsfähigen Substrats.

14. Verfahren nach einem der vorhergehenden Ansprüche, bei dem man das Substrat über einen Zeitraum bis zu 10 Minuten mit der Zusammensetzung in Berührung bringt.

15. Verfahren nach einem der vorhergehenden Ansprüche, bei dem man das Substrat bei einer Temperatur, die gleich dem oder kleiner als der Siedepunkt der Zusammensetzung ist, mit der Zusammensetzung in Berührung bringt.

16. Verfahren nach einem der vorhergehenden Ansprüche, bei dem man das Substrat mit der Zusammensetzung besprüht.

17. Verfahren nach einem der vorhergehenden Ansprüche, bei dem man das Substrat in die Zusammensetzung eintaucht.

18. Verfahren nach Anspruch 24, bei dem man das Substrat vollständig in die Zusammensetzung eintaucht.

19. Verfahren nach einem der vorhergehenden Ansprüche, bei dem man jegliche an exponierten Oberflächen des Substrats anhaftende Zusammensetzung durch Verdampfung entfernt.

20. Verfahren nach Anspruch 19, bei dem man die Zusammensetzung in weniger als 30 Sekunden durch Verdampfung entfernt.

21. Verfahren nach Anspruch 19 oder Anspruch 20, bei dem man die Verdampfung bei Normaldruck oder Unterdruck durchführt.

## Revendications

1. Procédé de séchage de la surface d'un substrat comprenant les étapes de mise en contact du substrat avec une composition comprenant du 1-chloro-3,3,3-trifluoropropène (HFCO-1233zd) et au moins un deuxième solvant choisi parmi des alcools du groupe constitué du méthanol, de l'éthanol ou de 1"isopropanol, et ensuite retrait de la composition à partir du substrat ;
dans lequel l'alcool est présent en une quantité de 1 à 50 pour cent en poids sur la base de la composition totale et le 1-chloro-3,3,3-trifluoropropène est présent en une quantité d'au moins 50 pour cent en poids sur la base du poids total de solvant présent dans la composition.

2. Procédé selon la revendication 1, dans lequel l'alcool est présent en une quantité de 1 à 45 pour cent en poids sur la base de la composition totale.

3. Procédé selon la revendication 2, dans lequel l'alcool est présent en une quantité de 1 à 4 pour cent en poids sur la base de la composition totale.

4. Procédé selon l'une quelconque des revendications précédentes dans lequel le 1-chloro-3,3,3-trifluoropropène (HFCO-1233zd) comprend cis-HFCO-1233zd, trans-HFCO-1233zd ou un mélange de ceux-ci.

5. Procédé selon l'une quelconque des revendications précédentes dans lequel le 1-chloro-3,3,3-trifluoropropène comprend trans-HFCO-1233zd.

6. Procédé selon l'une quelconque des revendications 1 à 4, dans lequel the 1-chloro-3,3,3-trifluoropropène comprend cis-HFCO-1233zd.

7. Procédé selon l'une quelconque des revendications précédentes, dans lequel l'au moins un deuxième solvant choisi parmi des alcools est le méthanol.

8. Procédé selon la revendication 1 pour sécher des métaux, des surfaces de verre et de céramique, de la silice et/ou de l'alumine.

9. Procédé selon la revendication 1, dans lequel la composition comprend en outre un tétrafluropropène.

10. Procédé selon la revendication 8, dans lequel la composition est destinée à déplacer l'eau depuis de l'acier inoxydable, des alliages d'aluminium et du laiton.

11. Procédé selon la revendication 8, dans lequel la composition est destinée à déplacer l'eau depuis du verre ou du verre borosilicate.

12. Procédé selon la revendication 8, dans lequel la composition est destinée à déplacer l'eau depuis des tranches de silicium.

13. Procédé selon l'une quelconque des revendications 1 à 12 pour sécher un substrat non absorbant.

14. Procédé selon l'une quelconque des revendications précédentes dans lequel le substrat est mis en contact avec la composition pendant jusqu'à 10 minutes.

15. Procédé selon l'une quelconque des revendications précédentes, dans lequel le substrat est mis en contact avec la composition à une température égal ou inférieure au point d'ébullition de la composition.

16. Procédé selon l'une quelconque des revendications précédentes, dans lequel le substrat est pulvérisé avec la composition.

17. Procédé selon l'une quelconque des revendications précédentes, dans lequel le substrat est immergé dans la composition.

18. Procédé selon la revendication 24, dans lequel le substrat est complètement immergé dans la composition.

19. Procédé selon l'une quelconque des revendications précédentes, dans lequel une composition quelconque adhérant à des surfaces exposées du substrat est éliminée par évaporation.

20. Procédé selon la revendication 19, dans lequel la composition est éliminée par évaporation en moins de 30 secondes.

21. Procédé selon la revendication 19 ou la revendication 20, dans lequel l'évaporation est conduite à pression atmosphérique ou subatmosphérique.
